Europäisches Patentamt

European Patent Office

Office européen des brevets

(19)

(11) Numéro de publication: **0 267 081**
**A1**

(12) **DEMANDE DE BREVET EUROPEEN**

(21) Numéro de dépôt: 87402237.9

(22) Date de dépôt: 08.10.87

(51) Int. Cl.⁴: **C 07 C 85/12**

(30) Priorité: 09.10.86 FR 8614044
09.10.86 FR 8614045

(43) Date de publication de la demande:
11.05.88 Bulletin 88/19

(84) Etats contractants désignés:
BE CH DE ES FR GB IT LI NL SE

(71) Demandeur: CECA S.A.
12-16 allée des Vosges
F-92400 Courbevoie (FR)

(72) Inventeur: Seffen, Mongi
4, rue El Basra Cité Béauséjour
Le Bardo 2000 (TN)

Forquy, Christian
Quartier Coos
64360 Monein (FR)

Barrault, Joel
La Brassalse
F-86240 Liguge (FR)

(54) Procédé perfectionné pour la production d'alkylamines et diméthylalkylamines à longue chaîne à partir d'esters.

(57) L'invention concerne un procédé de production d'amines à longue chaîne, et plus précisément, d'alkylamines primaires et de diméthyl-alkylamines, à partir des esters des acides carboxyliques correspondants. On fait réagir en une seule étape, l'ester avec l'ammoniac, l'hydrogène, et si nécessaire, du méthanol, en présence de catalyseurs spécifiques constitués d'oxyde de titane et de métaux actifs tels que le cuivre et le chrome, ou le cuivre, le chrome et le cobalt, sous des pressions d'environ de 50 à 100 bars.

EP 0 267 081 A1

**Description**

PROCEDE PERFECTIONNE POUR LA PRODUCTION D'ALKYLAMINES ET DE DIMETHYLALKYLAMINES A LONGUE CHAINE A PARTIR D'ESTERS

L'invention se rapporte à un procédé de synthèse directe d'amines grasses à partir des esters d'acides gras, par action de l'ammoniac et de l'hydrogène, en présence de catalyseurs mixtes de déshydratation/ hydrogénation; elle vise plus particulièrement à la production, avec un haut degré de sélectivité, d'amines primaires ou d'amines tertiaires diméthylées.

Les procédés industriels les plus utilisés pour la production d'amines à longue chaîne, c'est-à-dire dont la molécule contient de 10 à environ 24 atomes de carbone, sont encore largement basés sur la réaction classique de préparation du nitrile par réaction d'ammoniac sur l'acide gras et déshydratation, puis hydrogénation ultérieure du nitrile ainsi formé. On obtient ainsi l'alkylamine primaire à longue chaîne, à partir de laquelle on passe à la diméthylalkylamine par divers procédés, dont le plus anciennement connu est sans doute celui qui est adapté de la réaction de Leuckart, et qui consiste en une diméthylation par le système acide formique/formaldéhyde (par exemple brevet US 2366534), les procédés actuels utilisant plutôt la réaction du méthanol ou du formol en présence d'hydrogène.

On connaît aussi des procédés d'obtention d'alkylamines grasses à partir des acides gras et qui procèdent en une seule étape; ils mettent en jeu la réaction de l'hydrogène et de l'ammoniac ou d'amines légères en présence de catalyseurs métalliques, notamment au zinc-chrome ou au zinc-aluminium (brevet français no 1 549 655), au nickel avec divers cocatalyseurs (voir par exemple, le brevet d'Allemagne de l'Est no 110 487), ou encore à base de sulfures métalliques (brevet britanique no 1 135 915).

On trouve également décrits dans l'art antérieur, des procédés de fabrication d'amines mettant en oeuvre, comme matières premières, des triglycérides (brevet allemand no 1 288 595, brevet US no 3579 585). L'utilisation des esters comme matière de base pour l'obtention d'amines est citée notamment dans le brevet français no 1 598 720, le brevet des Etats-Unis d'Amérique no 2 223 305, le brevet britannique no 421 718, qui proposent de conduire les réactions d'amination sur catalyseurs mixtes à base de zinc ou d'alumine.

Les procédés en une étape qu'ils soient basés sur l'acide, le glycéride ou les esters, sont évidemment d'un meilleur intérêt industriel que les procédés traditionnels qui nécessitent deux étapes distinctes et deux catalyseurs distincts, l'un pour la déshydratation en nitrile, l'autre pour son hydrogénation, et un transfert de produit intermédiaire. Mais ils présentent aussi divers inconvénients, dont pour ceux qui néanmoins ont reçu un développement industriel, la nécessité de travailler à haute température et sous des pressions d'hydrogène généralement supérieures à 200 bars, et surtout de n'être pas sélectifs, c'est-à-dire de délivrer, en plus des alkylamines visées, des produits secondaires difficilement éliminables par les méthodes habituelles de séparation, notamment par distillation.

La présente invention pallie ces inconvénients. Elle permet l'obtention d'alkylamines à longue chaîne avec un très bon rendement et une excellente sélectivité grâce à un procédé fonctionnant en une seule étape, et qui consiste à faire réagir à des températures, qui en tout état de cause, n'excèdent pas 300° C, et sous des pressions qui restent comprises entre 50 et 100 bars, un ester d'acide gras à longue chaîne, avec de l'ammoniac, de l'hydrogène, et le cas échéant des réactifs destinés à améliorer la sélectivité de la réaction, en présence de catalyseurs mixtes du type déshydratant-hydrogénant.

Les esters d'acides gras à longue chaîne sont les esters méthyliques, éthyliques, propyliques ou butyliques d'acides gras comprenant de 10 à 24 atomes de carbone, et plus particulièrement des acides gras industriels issus du suif, de l'oléine, du colza, du palmiste ou du coprah. On préfère les esters méthyliques, qui sont devenus industriellement accessibles par méthanolyse des corps gras, et qui étant généralement liquides, sont d'une maniabilité fort appréciée au niveau des unités industrielles.

Les catalyseurs utiles pour la présente invention sont des catalyseurs de déshydratation-hydrogénation, résultant de la combinaison d'un métal et d'un oxyde acido-basique jouant de plus le rôle de support. On connaît beaucoup de catalyseurs de ce type, combinaisons de métaux nobles, de nickel, de cobalt, etc... et d'alumine, d'oxyde de zinc, d'oxyde de chrome ...

La demanderesse a trouvé dans l'association de l'oxyde de titane avec du cuivre et du cobalt, et surtout avec du cuivre, du chrome et du cobalt, un catalyseur hautement actif et très sélectif pour la conversion des méthylesters d'acides carboxyliques à longue chaîne en l'amine primaire ou en la diméthylamine correspondantes.

Les catalyseurs selon l'invention permettent de conduire la réaction dans des conditions de pression sensiblement plus basses que celles de l'art antérieur, et qui sont comprises entre 20 et 150 bars, et préférentiellement entre 50 et 100 bars, ce qui contribue fortement à réduire la formation de sous-produits lourds comme les dialkylamines. Ils contiennent de 2 à 80 % en poids de métaux (étant entendu que l'on désigne ici par métaux le cuivre, le cobalt et le chrome; cette acceptation du terme sera retenue dans toute la suite du document); on préfère cependant des teneurs comprises entre 10 et 50 % en poids.

Le cobalt y figure pour 2 à 30 % de l'ensemble du métal actif, les teneurs préférées étant de l'ordre de 10 %. Dans les système ternaires qui contiennent du chrome, ce métal peut être présent en proportion très variable qui peut aller de 1 à 50 % de la partie métallique.

On oriente de façon décisive la réaction vers la production de diméthylalkylamine en introduisant du méthanol dans le milieu réactionnel et en utilisant un catalyseur contenant de 10 à 25 % de chrome, en

2

préférant les teneurs voisines de 20 %.

Pour orienter la réaction vers la production d'amine primaire seule, on supprime le méthanol et on utilise un catalyseur contenant de 0,5 à 15 % de chrome, en préférant les teneurs basses de 1 à 1,5 %.

Ces catalyseurs sont obtenus par imprégnation, précipitation ou coprécipitation sur le support du métal pris sous la forme d'un sel hydrosoluble. Dans le mode de préparation par imprégnation, l'oxyde de titane est mis en suspension dans l'eau, et on lui ajoute le sel métallique tel quel ou sous forme de solution aqueuse. On évapore totalement la bouillie, et la poudre est séchée à l'étuve à l'air, à 100-150° C environ, puis calcinée, également à l'air, vers 350° C. Le résultat constitue le "précatalyseur", qui est la forme stockable du produit, et qui doit faire l'objet d'une activation, à effectuer au sein du réacteur. Dans le procédé par précipitation, on prépare comme précédemmment une barbotine d'oxyde de titane contenant les sels des métaux catalytiquement actifs, dont on précipite l'hydroxyde par l'ammoniac, en opérant à température d'environ 60 - 80° C, et à un pH maximum d'environ 7 à 8. Le solide est récupéré par filtration, puis séché et calciné comme dans le mode de préparation précédemment décrit.

L'invention est exposée ci-après dans sa réalisation de laboratoire, tranposable à l'échelle industrielle sans autres aménagements que ceux qui sont habituels en la matière.

On retrouvera dans tous les exemples qui vont suivre, un certain nombre d'éléments communs, qui ont trait à l'activation du catalyseur et à la conduite du réacteur pour la synthèse proprement dite. On les décrit ci-après.

La mise en oeuvre est effectuée dans un réacteur à lit fixe, de 200 ml environ de capacité, pouvant fonctionner sous pression et en continu. Après introduction du précatalyseur, on effectue une purge à l'azote, à raison de 50 l/h, à pression atmosphérique et à température ambiante.

On remplace ensuite l'azote par l'hydrogène, puis on élève progressivement la température, à raison de 4° C/min environ, jusqu'à ce que la température de réaction désirée soit atteinte. Ces conditions de température sont maintenues durant environ 12 heures.

La température est alors ajustée à la valeur désirée (selon la réaction, entre 250 et 350° C), et la pression augmentée à une valeur de l'ordre de 50 bars.

On introduit ensuite, par l'intermédiaire de pompes à membrane séparées, l'ester, le méthanol (si nécessaire) et l'ammoniac liquide, en contrôlant les débits de telle façon que le rapport ester/ammoniac/ hydrogène soit, dans les conditions standards pour la fabrication d'alkylamines primaires, 1/10/100, ou que le rapport ester/méthanol/ammoniac/hydrogène soit, dans les conditions standards, pour la fabrication des diméthylalkylamines, de 1/40/10/100, et que la vitesse spatiale du mélange réactionnel (millilitre par millilitre de catalyseur et par heure) soit d'environ 1/3 h$^{-1}$.

Ces conditions ne fixent que des ordres de grandeur, ajustables dans d'assez larges proportions pour tenir compte des conditions particulières des réactions.

Le mélange gaz-liquide sortant du réacteur est fractionné, la fraction liquide étant récupérée, les gaz étant soit rejetés, soit recyclés. La composition des produits de réaction est obtenue par analyse chromatographique en phase vapeur. Les meilleures conditions en sont :
- colonne chargée de chromosorb WAW (Johns Manville Corp.) imprégné de Carbowax 20 M (Johns Manville Corp.) et de potasse (2 %),
- gaz vecteur azote (30 ml/min),
- température du four à l'équilibre de 225° C.

Les étalonnages et les bilans réactionnels sont effectués à l'aide d'aniline comme étalon interne. Le procédé s'applique aussi bien à une synthèse en réacteur fermé, qu'à une synthèse en continu sur réacteur à lit fixe, auquel cas, les gaz non convertis issus du fractionnement du mélange sortant du réacteur sont recyclés.

On comprendra mieux l'invention à partir des exemples suivants qui décrivent des synthèses de dodécylamine ou de diméthyldodécylamine à partir de dodécanoate de méthyle, sans que ces descriptions restreignent la portée de l'invention à la conversion de cette seule matière première.

Exemple 1

On opère sur un catalyseur de composition Cu/Al$_2$O$_3$, 9,1/90,9 % obtenu par imprégnation d'alumine par le nitrate cuivrique. La réaction est effectuée à la température de 300° C, avec un rapport molaire ester/ammoniac/hydrogène de 1/10/100, et une vitesse spatiale du réactif de 0,33 h$^{-1}$.

La conversion est totale, mais la sélectivité est mauvaise, ainsi que le montre le bilan de composition des effluents liquides :

Dodécylamine          72,0 %
N-méthyldodécylamine          16,0 %
N, N-diméthyldodécylamine          2,5 %
Dodécylamide          8,0 %
Dodécylnitrile          1,0 %
Autres          0,5 %

Exemple 2

Si on essaye d'orienter la réaction vers la production de diméthylamine en utilisant un flux de réactif contenant du méthanol, on obtient bien une augmentation du taux d'amine tertiaire, mais avec une tout aussi mauvaise sélectivité, ainsi qu'en témoigne la composition des effluents liquides ci-après obtenue sur catalyseur Cu/Al$_2$O$_3$ 17,8/82,2 %, avec un rapport ester/méthanol/ammoniac/hydrogène de 1/40/10/100, une vitesse spatiale de 0,17 h$^{-1}$, et à la température de 250° C.

Dodécylamine        29,2 %
N-méthyldodécylamine        34,7 %
N, N-diméthyldodécylamine        15,6 %
Dodécylamide        9,1 %
Dodécylnitrile        11,4 %

Exemple 3

La synthèse est conduite dans les conditions de l'exemple 1 sur catalyseur Cu/ZnO, 9/91. L'ester est converti à raison de 90 % mais la composition de la fraction convertie :

Dodécylamine        75,0 %
N-méthyldodécylamine        6,7 %
N, N-diméthyldodécylamine        1,5 %
Dodécylamide        14,0 %
Dodécylnitrile        2,5 %
Autres amines        0,3 %

témoigne d'un mauvais rendement en amine primaire, surtout dû à la formation d'amide.

Exemple 4

L'opération, dont on espère un bon rendement en amine primaire, est exécutée dans les conditions de l'exemple 1 sur un catalyseur du type chromite de cuivre, de composition Cu/Cr$_2$O$_3$, 40/60. On a opéré à deux températures de 300 et 350° C.

Le bilan de composition de la partie des effluents correspondant à l'ester effectivement converti est le suivant :

| Température | 300° C | 350° C |
|---|---|---|
| Dodécylamine | 67,0 % | 74,0 % |
| N-méthyldodécylamine | 3,4 % | 4,3 % |
| N, N-diméthyldodécylamine | 15,4 % | 14,5 % |
| Dodécylamide | 9,7 % | 1,0 % |
| Dodécylnitrile | 3,6 % | 4,7 % |
| Autres amines | 0,9 % | 1,5 % |

Dans les deux cas, la sélectivité est mauvaise ou très médiocre, bien que les taux de conversion atteignent respectivement 95 % et 100 %.

Exemple 5

On tente ici d'obtenir la diméthyldodécylamine en opérant, également sur catalyseur Cu/Cr$_2$O$_3$, 40/60 dans les conditions voisines de celles de l'exemple 2, avec deux rapports Hydrogène/ester de 100 et 400. On obtient :

| H$_2$/ester | 100 | 400 |
|---|---|---|
| N-méthyldodécylamine | 7,4 % | 28,2 % |
| N, N-diméthyldodécylamine | 30,2 % | 43,2 % |
| Dodécylamine | 1,5 % | 4,9 % |
| Dodécylamide | 44,2 % | 17,0 % |
| Dodécylnitrile | 3,6 % | 2,5 % |
| Autres amines | 13,1 % | 10,2 % |

Cette association cuivre-oxyde de chrome est intéressante en ce qu'elle conduit à un rendement déjà non négligeable en méthylalkylamines, mais au prix de teneurs inacceptables en amide et en dialkylamines, et avec une sélectivité d'autant plus mauvaise que le rendement global en méthylamines est meilleur.

Exemple 6

La réaction est effectuée selon l'invention en présence de catalyseur préparé par coimprégnation et de composition Cu/Co/TiO$_2$, 20/2,5/77,5. La température de réaction est de 250° C. Toutes les autres conditions opératoires sont identiques à celles de l'exemple 1. On obtient :

Dodécylamine 86,2 %
N-méthyldodécylamine 2,1 %
N, N-diméthyldodécylamine 0,9 %
Dodécylamide Non mesurable
Dodécylnitrile Non mesurable
Dodécane 1,7 %
Autres amines 9,1 %

Exemple 7

La réaction est effectuée selon l'invention à l'aide d'un catalyseur mixte Cu/Cr/Co/TiO$_2$, obtenu par coimprégnation d'oxyde de titane, et de composition 20/1,5/1,5/77. La température de réaction est de 250°. Toutes les autres conditions sont celles de l'exemple 1. On obtient :

Dodécylamine 83,0 %
N-méthyldodécylamine 4,1 %
N, N-diméthyldodécylamine 0,5 %
Dodécylamide 1,0 %
Dodécylnitrile 0,7 %
Dodécane 8,0 %
Autres amines 2,7 %

Exemple 8

La réaction est conduite selon l'invention sur catalyseur Cu/Cr/Co/TiO$_2$, de composition pondérale 15,1/1,1/2,6/81,2, préparé par mélange mécanique de catalyseurs d'imprégnation Co/TiO$_2$ et Cu/Cr/TiO$_2$. L'effluent liquide a la composition suivante :

Dodécylamine 74,5 %
N-méthyldodécylamine 1,0 %
N, N-diméthyldodécylamine 1,0 %
Dodécylamide 1,0 %
Dodécylnitrile 1,0 %
Dodécane 16,4 %
Autres 5,1 %

On voit sur cet exemple, comme sur les deux exemples précédents, que l'association de l'oxyde de titane avec des compositions métalliques cuivre-cobalt ou cuivre-chrome-cobalt permet d'obtenir une conversion pratiquement totale de l'ester, et que l'alkylamine à longue chaîne formée contient très peu d'homologues méthylés. Les hydrocarbures peuvent constituer une part non négligeable des sous-produits de la réaction; ils ne sont généralement pas gênants dans les applications ultérieures de l'amine primaire, et de toute façon, sont facilement séparables.

Exemple 9

On oriente ici la réaction vers la production de la diméthylalkylamine, avec un catalyseur selon l'invention à base d'oxyde de titane et de ternaire Cu/Cr/Co fortement enrichi en chrome et en utilisant un effluent contenant du méthanol.

La composition pondérale du catalyseur est ici pour $TiO_2$/Cu/Cr/Co de 55,6/22,1/20,1/2,2. Les conditions de la réaction sont un rapport ester/méthanol/ammoniac/hydrogène de 1/40/10/400, une température de 250° C, et une vitesse spatiale de 0,33 $h^{-1}$. On obtient ainsi une conversion quasi-totale de l'ester en diméthyldodécylamine. La composition des effluents s'établit à :

N-méthyldodécylamine     4 %
N, N-diméthyldodécylamine     93 %
Dodécylamine     1,5 %
Dodécylamide     0,5 %
Dodécylnitrile     Non mesurable
Autres amines     Non mesurable
Dodécane     1 %

**Revendications**

1. Procédé pour la production d'alkylamines primaires et de diméthylalkylamines, à chaîne alkyle constituée par un reste hydrocarboné comprenant de 10 à 24 atomes de carbone, fonctionnant par réaction en une seule étape d'esters d'acides carboxyliques à longue chaîne et d'un effluent contenant de l'ammoniac et de l'hydrogène, en présence d'un catalyseur mixte du type oxyde-métal, caractérisé en ce que le catalyseur est constitué d'une part d'oxyde de titane, et d'autre part de cuivre ou de cuivre et de chrome associés au cobalt.

2. Procédé selon la revendication 1, caractérisé en ce que le catalyseur contient de 2 à 80 % en poids de métaux du groupe constitué par le cuivre, le chrome et le cobalt.

3. Procédé selon les revendications 1 et 2, caractérisé en ce que le catalyseur contient de 10 à 50 % en poids de métaux du groupe constitué par le cuivre, le chrome et le cobalt.

4. Procédé selon les revendications 1 à 3, caractérisé en ce que l'ester d'acide carboxylique à longue chaîne est un ester méthylique.

5. Procédé selon la revendication 4, pour la production préférée de diméthylalkylamines, caractérisé en ce que l'effluent réactif contient, outre l'ammoniac et l'hydrogène, du méthanol et que la proportion pondérale de chrome dans la catalyseur est comprise entre 10 et 25 %.

6. Procédé selon la revendication 4 pour la production préférée de monoalkylamines, caractérisé en ce que l'effluent contient l'ammoniac et l'hydrogène à l'exclusion de méthanol, et que la proportion pondérale de chrome dans le catalyseur est comprise entre 0,5 et 15 %.

7. Catalyseur de type oxyde-métal, pour la production d'alkylamines primaires et de diméthylalkylamines à chaîne hydrocarbonée contenant de 10 à 24 atomes de carbone, par réaction d'esters d'acides carboxyliques à longue chaîne et d'un effluent contenant de l'ammoniac et de l'hydrogène, caractérisé en ce qu'il contient de l'oxyde de titane et du cuivre, du chrome et du cobalt.

8. Catalyseur selon la revendication 7, caractérisé en ce qu'il contient de 2 à 80 % en poids de cuivre, chrome et cobalt.

9. Catalyseur selon la revendication 8, caractérisé en ce qu'il contient de 0,5 à 25 % de chrome.

Office européen des brevets

# RAPPORT DE RECHERCHE EUROPEENNE

Numero de la demande

EP 87 40 2237

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | GB-A- 421 718 (I.G. FARBENINDUSTRIE AG)<br>* Page 9, lignes 38-65; exemples *<br>--- | 1-9 | C 07 C 85/12 |
| D,A | US-A-2 223 303 (W.A. LAZIER)<br>* Exemple VI *<br>--- | 1 | |
| D,A | FR-A-1 598 720 (HENKEL & CIE)<br>* Exemples; résumé *<br>--- | 1 | |
| D,A | FR-A-1 549 655 (HENKEL & CIE)<br>* Exemples; résumé *<br>----- | 1 | |

DOMAINES TECHNIQUES RECHERCHES (Int. Cl.4)

C 07 C 85/00

Le présent rapport a été établi pour toutes les revendications

| Lieu de la recherche | Date d'achèvement de la recherche | Examinateur |
|---|---|---|
| LA HAYE | 15-01-1988 | PAUWELS G.R.A. |

EPO FORM 1503 03.82 (P0402)